# EUROPEAN PATENT SPECIFICATION

(11) **EP 3 676 306 B1**
(45) Date of publication and mention of the grant of the patent: **25.05.2022**
(21) Application number: 18762753.4
(22) Date of filing: 28.08.2018
(51) Int. Cl.: C08G 18/48, A61B 5/145, C08G 18/75, C08L 75/08, C12Q 1/00

(54) **SYNTHETIC MEMBRANE COMPOSITION COMPRISING POLYURETHANE BLEND**
SYNTHETISCHE MEMBRANZUSAMMENSETZUNG EINER POLYURETHANMISCHUNG
COMPOSITION DE MEMBRANE SYNTHÉTIQUE COMPRENANT UN MÉLANGE DE POLYURÉTHANE

(30) Priority: 28.08.2017 US 201762550922 P; 28.08.2017 US 201762550929 P; 28.08.2017 US 201762550937 P; 15.09.2017 EP 17191475; 15.09.2017 EP 17191476; 15.09.2017 EP 17191477
(43) Date of publication of application: 08.07.2020
(73) Proprietor: DSM IP Assets B.V., 6411 TE Heerlen (NL)
(72) Inventor: AL-RASHID, Jennifer, Exton, Pennsylvania 19341 (US); ZUPANCICH, John, Exton, Pennsylvania 19341 (US); CHALASANI, Durga Prasad, Exton, Pennsylvania 19341 (US); SUGIYAMA, Chad, Exton, Pennsylvania 19341 (US)
(74) Representative: DSM Intellectual Property
(86) International application number: PCT/US2018/048312
(87) International publication number: WO 2019/046281

(56) References cited:
- US-A- 5 589 563
- US-A1- 2006 258 761
- US-B2- 7 226 978

## Description

### Field

The disclosed inventions pertain to compositions that may be useful for forming synthetic membranes, methods of forming membranes therefrom, and membranes formed therefrom.

### Background

A sensor may comprise an analyte diffusion-limiting membrane. Without an analyte diffusion-limiting membrane, a sensor becomes saturated quickly and at low analyte concentrations. Ideally, a sensor has sufficient oxygen for adequate operation, but saturation of the target analyte at the sensor surface is prevented. An oxygen permeable membrane that restricts analyte flux to the sensing layer is thus often required. Preferred diffusion-limiting membranes are mechanically strong, biocompatible, minimize protein adsorption, have sufficient oxygen diffusivity, and are easily manufactured.

Synthetic membranes formed from polyurethanes are known. Polyurethanes have been chosen due to their ability to form films when blended with a range of solvents and ability to regulate the flux of analytes to sensors.

For instance, US5589563 discloses the casting of membranes from polyurethanes comprising surface modifying endgroups. A polyurethane with a surface modifying endgroups is a polyurethane comprising one or more endgroups at the terminal ends of the backbone of the polyurethane. The surface modifying endgroups and backbone are such that the surface activity of such a polyurethane reflects the surface activity of the surface modifying endgroups rather than the backbone.

Further techniques for forming membranes from polyurethanes are disclosed in US7226978. A membrane formed from a blend of amphiphilic copolymer and hydrophobic polymer is disclosed. It is stated that the blend allows membranes having hydrophilic domains that control the diffusion of an analyte therethrough dispersed in a hydrophobic matrix. Polyurethanes may be employed as the amphiphilic copolymer or hydrophobic polymer.

Another technique for forming membranes is disclosed in US7157528 and US7687586. In these documents a biocompatible multipolymer is disclosed. The multipolymer comprises a hydrophilic soft segment and an oxygen-permeable soft segment.

Further techniques for forming membranes from polyurethanes are disclosed in US8255032. A membrane comprising a blend of silicone-containing polyurethane with hydrophilic polymer is disclosed. The hydrophilic polymer may be polyvinylpyrrolidone, polyhydroxyethyl methacrylate, polyvinylalcohol, polyacrylic acid, polyethers, and copolymers thereof.

In US2006/0258761 membrane systems incorporating silicone polymers are described for use in implantable analyte sensors. Some layers of the membrane system may comprise a blend of a silicone polymer with a hydrophilic polymer, for example, a triblock poly(ethylene oxide)-poly(propylene oxide)-poly(ethylene oxide) polymer.

Despite these documents, there is still a need for compositions for forming synthetic membranes that yield suitable mechanical properties and film quality, in a highly reproducible process.

### Summary

Known compositions for forming synthetic membranes may be deficient in desired performance and processing characteristics. For example, forming films from a blend of a hydrophilic polymer, such as PVP, and a hydrophobic polyurethane may present difficulty in reproducibility due to microphase separation of the dissimilar polymers. Furthermore, such membranes may suffer from reduced analyte diffusivity. These difficulties can be overcome by using strong, high boiling point organic solvents, such as dimethylacetamide (DMAc) or dimethylformamide (DMF), but this may present health and safety concerns because such solvents are not easily removed from the polymers. Furthermore, certain solvents may dissolve polymer layers on a sensor, restricting their use in certain sensor-related applications.

In accordance with an embodiment, a composition for forming a membrane comprises from 0.5 wt% to 10 wt% of a polyurethane component and from 90 to 99.5 wt% of a solvent, the polyurethane component comprising a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane, wherein the amphiphilic polyurethane comprises a first amphiphilic polyurethane or a second amphiphilic polyurethane, and
a. the first amphiphilic polyurethane comprises the reaction product of:
   i. an aliphatic diisocyanate;
   ii. a hydrophilic polymer diol;
   iii. a hydrophobic poly(alkylene oxide) diol; and
   iv. a chain extender;
b. the second amphiphilic polyurethane comprises the reaction product of:
   i. an aliphatic diisocyanate;
   ii. a copolymer diol comprising a hydrophilic polymer and a hydrophobic poly(alkylene oxide); and
   iii. a chain extender;
c. the hydrophobic polyurethane comprises the reaction product of:
   i. an aliphatic diisocyanate;
   ii. a hydrophobic poly(alkylene oxide) diol; and
   iii. a chain extender;
   wherein the amphiphilic polyurethane and the hydrophobic polyurethane are substantially devoid of siloxane moieties.

In accordance with an embodiment, a membrane comprises a polyurethane component, the polyurethane component comprising a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane, wherein the amphiphilic polyurethane comprises a first amphiphilic polyurethane or a second amphiphilic polyurethane, and
a. the first amphiphilic polyurethane comprises the reaction product of:
   i. an aliphatic diisocyanate;
   ii. a hydrophilic polymer diol;
   iii. a hydrophobic poly(alkylene oxide) diol; and
   iv. a chain extender;
b. the second amphiphilic polyurethane comprises the reaction product of:
   i. an aliphatic diisocyanate;
   ii. a copolymer diol comprising a hydrophilic polymer and a hydrophobic poly(alkylene oxide); and
   iii. a chain extender;
c. the hydrophobic polyurethane comprises the reaction product of:
   i. an aliphatic diisocyanate;
   ii. a hydrophobic poly(alkylene oxide) diol; and
   iii. a chain extender;

   wherein the amphiphilic polyurethane and the hydrophobic polyurethane are substantially devoid of siloxane moieties, and
   wherein the membrane has a residual solvent content of less than 50 ppm after drying the membrane in a convection oven at 50 °C for 16 hours after forming the membrane.

The compositions, methods, membranes, and articles disclosed herein may exhibit benefits in film formation, reproducibility, mechanical properties, anti-fouling, health and safety, improved compatibility with sensors, and/or solvent removal.

### Brief Description of the Drawings

Fig. 1 is a SEM image of a film formed in Example 1.
Fig. 2 is a SEM image of a film formed in Example 1.

### Detailed Description

In accordance with an embodiment, a composition for forming a membrane comprises from 0.5 wt% to 10 wt% of a polyurethane component and from 90 to 99.5 wt% of a solvent, the polyurethane component comprising a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane, wherein the amphiphilic polyurethane comprises a first amphiphilic polyurethane or a second amphiphilic polyurethane, and
a. the first amphiphilic polyurethane comprises the reaction product of:
   i. an aliphatic diisocyanate;
   ii. a hydrophilic polymer diol;
   iii. a hydrophobic poly(alkylene oxide) diol; and
   iv. a chain extender;
b. the second amphiphilic polyurethane comprises the reaction product of:
   i. an aliphatic diisocyanate;
   ii. a copolymer diol comprising a hydrophilic polymer and a hydrophobic poly(alkylene oxide); and
   iii. a chain extender;
c. the hydrophobic polyurethane comprises the reaction product of:
   i. an aliphatic diisocyanate;
   ii. a hydrophobic poly(alkylene oxide) diol; and
   iii. a chain extender;
   wherein the amphiphilic polyurethane and the hydrophobic polyurethane are substantially devoid of siloxane moieties.

In accordance with an embodiment, a membrane comprises a polyurethane component, the polyurethane component comprising a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane, wherein the amphiphilic polyurethane comprises a first amphiphilic polyurethane or a second amphiphilic polyurethane, and
a. the first amphiphilic polyurethane comprises the reaction product of:
   i. an aliphatic diisocyanate;
   ii. a hydrophilic polymer diol;
   iii. a hydrophobic poly(alkylene oxide) diol; and
   iv. a chain extender;
b. the second amphiphilic polyurethane comprises the reaction product of:
   i. an aliphatic diisocyanate;
   ii. a copolymer diol comprising a hydrophilic polymer and a hydrophobic poly(alkylene oxide); and
   iii. a chain extender;
c. the hydrophobic polyurethane comprises the reaction product of:
   i. an aliphatic diisocyanate;
   ii. a hydrophobic poly(alkylene oxide) diol; and
   iii. a chain extender;

   wherein the amphiphilic polyurethane and the hydrophobic polyurethane are substantially devoid of siloxane moieties, and
   wherein the membrane has a residual solvent content of less than 50 ppm after drying the membrane in a convection oven at 50 °C for 16 hours after forming the membrane.

By a reaction product it is meant that the diisocyanate and diol(s), and optionally the chain extender, are engaged in a simultaneous or sequential chemical reaction. For example, a reaction product of a diisocyanate, a diol, and a chain extender is formed i) when the diisocyanate, diol, and chain extender are all reacted together in a single solution, or ii) when a pre-polymer is first formed by reacting the diisocyanate and the diol, and then this prepolymer is subsequently reacted with the chain extender.

The disclosed compositions, membranes, and sensors may have advantages over the prior art in terms of mechanical properties, such as modulus, tensile strength, elongation, or durability, analyte permeability, oxygen permeability, isotropy of mechanical properties, surface quality, use with a wider range of solvents, such as THF, process reproducibility, process speed, health and safety concerns, such as easier or more expedient removal of residual solvent.

The amphiphilic polyurethane and the hydrophobic polyurethane comprise a backbone that comprises the reaction product of an aliphatic diisocyanate, a diol, and a chain extender. In an embodiment, the amphiphilic polyurethane, the hydrophobic polyurethane, or both are aliphatic. In an embodiment, the polyurethane consists of a backbone that comprises the reaction product of an aliphatic diisocyanate, a polymeric aliphatic diol, and, optionally, a chain extender. In an embodiment, the amphiphilic polyurethane and/or the hydrophobic polyurethane further comprise an endgroup. In an embodiment, the amphiphilic polyurethane and/or the hydrophobic polyurethane is linear. In an embodiment, the amphiphilic polyurethane and/or the hydrophobic polyurethane is branched.

In an embodiment, the amphiphilic polyurethane and the hydrophobic polyurethane are substantially devoid of siloxane moieties or are devoid of siloxane moieties. In an embodiment, the composition or membrane is devoid of siloxane.

Descriptions of the various components of the compositions and reactants from which the components in the compositions are formed, are described in the following paragraphs. Unless stated otherwise, descriptions of embodiments involving components that may be used to form both the amphiphilic polyurethane and the hydrophobic polyurethane are specified are meant to disclose the situations where the component is as described in the amphiphilic polyurethane, the hydrophobic polyurethane, or both, as applicable. Furthermore, when it is specified that the amphiphilic polyurethane comprises a feature, it is disclosed that the first amphiphilic polyurethane comprises the feature, the second amphiphilic polyurethane comprises the feature, or both the first and second amphiphilic polyurethane comprise the feature. For example, where it is specified that the endgroup is linear, the situation is disclosed where the first amphiphilic polyurethane comprises a linear endgroup, the second amphiphilic polyurethane comprises a linear endgroup, both the first and second amphiphilic polyurethane comprise a linear endgroup, the hydrophobic polyurethane comprises a linear endgroup, and any combination of the preceding.

### Aliphatic Diisocyanate Component

Both the amphiphilic polyurethane and the hydrophobic polyurethane comprise the residue of an aliphatic diisocyanate. In an embodiment, the diisocyanate comprises an average of at least 1.9 isocyanate groups per molecule and an average of less than 2.7 isocyanate groups per molecule.

In an embodiment, the diisocyanate comprises hexamethylene diisocyanate, dicyclohexylmethane 4,4'-diisocyanate, isophorone diisocyanate, or a mixture thereof. In an embodiment, the diisocyanate consists of hexamethylene diisocyanate, dicyclohexylmethane 4,4'-diisocyanate, isophorone diisocyanate, or a mixture thereof.

In an embodiment, the molecular weight of the diisocyanate is from 100 to 500 g/mol. In an embodiment, the molecular weight of the diisocyanate is from 150 to 260 g/mol.

In an embodiment, the amphiphilic polyurethane comprises at least 10 wt%, at least 20 wt%, at least 25 wt%, at least 30 wt%, at least 35 wt%, or at least 40 wt% of a diisocyanate, based on the total weight of the formulation from which the amphiphilic polyurethane is formed. In an embodiment, the amphiphilic polyurethane comprises at most 50 wt%, at most 40 wt%, at most 35 wt%, at most 30 wt%, at most 25 wt%, or at most 20 wt% of a diisocyanate, based on the total weight of the formulation from which the amphiphilic polyurethane is formed. In an embodiment, the hydrophobic polyurethane comprises at least 10 wt%, at least 20 wt%, at least 25 wt%, at least 30 wt%, at least 35 wt%, or at least 40 wt% of a diisocyanate, based on the total weight of the formulation from which the hydrophobic polyurethane is formed. In an embodiment, the hydrophobic polyurethane comprises at most 50 wt%, at most 40 wt%, at most 35 wt%, at most 30 wt%, at most 25 wt%, or at most 20 wt% of a diisocyanate, based on the total weight of the formulation from which the hydrophobic polyurethane is formed.

### Diol

The amphiphilic polyurethane and hydrophobic polyurethane both comprises the residue of a diol. The amphiphilic polyurethane comprises the residue of both a hydrophilic diol and a hydrophobic poly(alkylene oxide) diol, or the residue of a copolymer diol comprising a hydrophilic polymer and a hydrophobic poly(alkylene oxide). The hydrophobic polyurethane comprises the residue of a hydrophobic poly(alkylene oxide) diol.

A hydrophobic poly(alkylene oxide) diol is a poly(alkylene oxide) diol that tends to repel and not absorb water, or to not be dissolvable in water. In an embodiment, the hydrophobic poly(alkylene oxide) diol comprises poly(propylene oxide) diol, poly(tetramethylene oxide) diol, or a diol that comprises a copolymer of poly(propylene oxide) and poly(tetramethylene oxide). In an embodiment, the hydrophobic poly(alkylene oxide) diol has a number average molecule weight (Mn) of at least 250 g/mol, at least 500 g/mol, at least 750 g/mol, at least 1000 g/mol, or at least 1500 g/mol. In an embodiment, the hydrophobic poly(alkylene oxide) diol has a Mn of at most 10000 g/mol, at most 8000 g/mol, at most 6000 g/mol, at most 5000 g/mol, at most 3500 g/mol, or at most 2500 g/mol.

In contrast, a hydrophilic diol is a diol that tends to attract and absorb water, or be dissolvable in water. In an embodiment, the hydrophilic diol is a hydrophilic poly(alkylene oxide) diol. In an embodiment, the hydrophilic poly(alkylene oxide) diol is a poly(ethylene oxide) diol. In an embodiment, the hydrophilic diol has a number average molecule weight (Mn) of at least 250 g/mol, at least 500 g/mol, at least 750 g/mol, at least 1000 g/mol, or at least 1500 g/mol. In an embodiment, the hydrophilic diol has a Mn of at most 10,000 g/mol, at most 8000 g/mol, at most 6000 g/mol, at most 5000 g/mol, at most 3500 g/mol, or at most 2500 g/mol.

In an embodiment, the amphiphilic polyurethane comprises a second amphiphilic polyurethane comprising a copolymer diol comprising a hydrophilic polymer and a hydrophobic poly(alkylene oxide). In an embodiment, the amphiphilic polyurethane comprises a second amphiphilic polyurethane comprising a copolymer diol comprising a hydrophilic poly(alkylene oxide) and a hydrophobic poly(alkylene oxide). In an embodiment, the copolymer diol comprises a copolymer of poly(ethylene oxide) with poly(propylene oxide) and/or poly(tetramethylene oxide). In an embodiment, the copolymer diol comprising a hydrophilic polymer and a hydrophobic poly(alkylene oxide) has a number average molecule weight (Mn) of at least 500 g/mol, at least 750 g/mol, at least 1000 g/mol, or at least 1500 g/mol, or at least 2000 g/mol. In an embodiment, the copolymer diol comprising a hydrophilic polymer and a hydrophobic poly(alkylene oxide)has a Mn of at most 10000 g/mol, at most 8000 g/mol, at most 6000 g/mol, at most 5000 g/mol, at most 3500 g/mol, or at most 2500 g/mol.

The residues of further diols may be present in the amphiphilic polyurethane or hydrophobic polyurethane. In an embodiment, the amphiphilic polyurethane or hydrophobic polyurethane comprises the residue of a poly(isobutylene) diol, a polyester diol, for example a polyester diol formed from adipic acid or isophtalic acid and a monomeric diol, an alkane diol, such as a hydrogenated polybutadiene diol or a polyethylene diol, or a polycarbonate diol.

In an embodiment, the amphiphilic polyurethane comprises at least 30 wt%, at least 35 wt%, at least 40 wt%, at least 45 wt%, or at least 50 wt%, based on the total weight of the formulation from which the amphiphilic polyurethane is formed, of a diol. In an embodiment, the amphiphilic polyurethane comprises at most 85 wt%, at most 80 wt%, at most 75 wt%, at most 70 wt%, at most 65 wt%, or at most 60 wt%, based on the total weight of the formulation from which the amphiphilic polyurethane is formed, of a diol.

In an embodiment, the amphiphilic polyurethane comprises at least 30 wt%, at least 35 wt%, at least 40 wt%, at least 45 wt%, or at least 50 wt%, based on the total weight of the formulation from which the amphiphilic polyurethane is formed, of a hydrophilic polymer diol, a hydrophobic poly(alkylene oxide) diol, and/or a copolymer diol comprising a hydrophilic polymer and a hydrophobic poly(alkylene oxide). In an embodiment, the amphiphilic polyurethane comprises at most 85 wt%, at most 80 wt%, at most 75 wt%, at most 70 wt%, at most 65 wt%, or at most 60 wt%, based on the total weight of the formulation from which the amphiphilic polyurethane is formed, of a hydrophilic polymer diol, a hydrophobic polymer diol, and/or a copolymer diol comprising a hydrophilic polymer and a hydrophobic poly(alkylene oxide).

In an embodiment, the hydrophobic polyurethane comprises at least 30 wt%, at least 35 wt%, at least 40 wt%, at least 45 wt%, or at least 50 wt%, based on the total weight of the formulation from which the hydrophobic polyurethane is formed, of a diol. In an embodiment, the hydrophobic polyurethane comprises at most 85 wt%, at most 80 wt%, at most 75 wt%, at most 70 wt%, at most 65 wt%, or at most 60 wt%, based on the total weight of the formulation from which the hydrophobic polyurethane is formed, of a diol.

In an embodiment, the hydrophobic polyurethane comprises at least 30 wt%, at least 35 wt%, at least 40 wt%, at least 45 wt%, or at least 50 wt%, based on the total weight of the formulation from which the hydrophobic polyurethane is formed, of a hydrophobic poly(alkylene oxide) diol. In an embodiment, the hydrophobic polyurethane comprises at most 85 wt%, at most 80 wt%, at most 75 wt%, at most 70 wt%, at most 65 wt%, or at most 60 wt%, based on the total weight of the formulation from which the hydrophobic polyurethane is formed, of a hydrophobic poly(alkylene oxide). In an embodiment, the polyurethane comprises at least 20 wt%, at least 30 wt%, at least 40 wt%, at least 50 wt%, or at least 60 wt% of the residue of a polymeric aliphatic diol, based on the total weight of the polyurethane. In an embodiment, the polyurethane comprises at most 80 wt%, at most 70 wt%, at most 60 wt%, or at most 50 wt% of the residue of a polymeric aliphatic diol, based on the total weight of the polyurethane.

### Chain Extender

Both the amphiphilic polyurethane and the hydrophobic polyurethane comprise the residue of a chain extender. A chain extender is an alkane diol having from 2 to 20 carbon atoms, wherein one or more carbon atoms may be substituted with oxygen. In an embodiment, the chain extender has a molecular weight of at least 60 g/mol, at least 70 g/mol, at least 80 g/mol, at least 90 g/mol, or at least 100 g/mol. In an embodiment, the chain extender has a molecular weight of at most 500 g/mol, at most from 400 g/mol, at most 300 g/mol, at most 200 g/mol, or at most 150 g/mol. In an embodiment, the chain extender comprises ethylene glycol, diethylene glycol, propylene glycol, dipropylene glycol, 1,3-propanediol, 1,4-butanediol, 1,5-pentanediol, 1,6-hexanediol, or 1,8-octanediol.

In an embodiment, the polyurethane is formed from a formulation that comprises at least 1 wt%, at least 2 wt%, at least 5 wt%, at least 8 wt%, or at least 10 wt% of a chain extender, based on the total weight of the formulation. In an embodiment, the polyurethane is formed from a formulation that comprises at most 20 wt%, at most 15 wt%, at most 12 wt%, at most 10 wt%, at most 8 wt%, or at most 5 wt%, of a chain extender, based on the total weight of the formulation. In an embodiment, the polyurethane comprises at least 1 wt%, at least 2 wt%, at least 5 wt%, at least 8 wt%, or at least 10 wt% of the residue of a chain extender, based on the total weight of the polyurethane. In an embodiment, the polyurethane comprises at most 20 wt%, at most 15 wt%, at most 12 wt%, at most 10 wt%, at most 8 wt%, or at most 5 wt%, of the residue of a chain extender, based on the total weight of the polyurethane.

### Endgroups

In an embodiment, the amphiphilic polyurethane, the hydrophobic polyurethane, or both further comprise one or more endgroups. An endgroup is a moiety present at a terminal end of a molecule. In an embodiment, the amphiphilic polyurethane, the hydrophobic polyurethane, or both are linear and further comprise an endgroup at each terminus of the backbone. In an embodiment, the endgroup is linear. In an embodiment, the endgroup is branched. In an embodiment, the amphiphilic polyurethane, the hydrophobic polyurethane, or both further comprises an average of at least 0.1 endgroups, at least 0.25 endgroups, at least 0.5 endgroups, at least 1 endgroup, at least 1.5 endgroups, at least 1.8 endgroups, or at least 2 endgroups. In an embodiment, the amphiphilic polyurethane, the hydrophobic polyurethane, or both comprise an average of at most 4 endgroups an average of at most 2 endgroups, or an average of at most 2 endgroups. In an embodiment, the amphiphilic polyurethane, the hydrophobic polyurethane, or both further comprise am average of about 2 endgroups.

An endgroup may be formed by reacting a terminal isocyanate group present after forming the polymer backbone with a coreactive group on a monofunctional moiety. For instance, a terminal isocyanate group may be reacted with 1-octanol or octylamine to form a C₈ alkyl endgroup. Endgroups may also result from the inclusion of chain stoppers, such as monofunctional alcohols, in a formulation used in the formation of a polyurethane. For instance, a formulation for forming a polyurethane may comprise a diisocyanate, a polymeric aliphatic diol, a chain extender, and a monofunctional alcohol.

In an embodiment, the endgroup comprises a hydrophobic poly(alkylene oxide), a hydrophilic poly(alkylene oxide), a copolymer comprising a hydrophilic poly(alkylene oxide) and a hydrophobic poly(alkylene oxide), C₂-C₂₀ alkyl, C₂-C₁₆ fluoroalkyl, C₂-C₁₆ fluoroalkyl ether, or copolymers thereof. In an embodiment, the hydrophilic poly(alkylene oxide) is poly(ethylene oxide). In an embodiment, the hydrophobic poly(alylene oxide) is poly(propylene oxide) or poly(tetramethylene oxide). In an embodiment, the endgroup comprises a hydrophobic poly(alkylene oxide), a hydrophilic poly(alkylene oxide), a copolymer comprising a hydrophilic poly(alkylene oxide) and a hydrophobic poly(alkylene oxide), C₂-C₂₀ alkyl, C₂-C₁₆ fluoroalkyl, C₂-C₁₆ fluoroalkyl ether, or copolymers thereof. In an embodiment, the amphiphilic polyurethane comprises an endgroup and the endgroup comprises poly(ethylene oxide). Such endgroups may be formed with monofunctional alcohols, including carbinols, or amines of the foregoing.

In an embodiment, the endgroup is monomeric and has a molecular weight of 200 g/mol or more, 300 g/mol or more, or 500 g/mol or more. In an embodiment, the endgroup is monomeric and has a molecular weight of 1,000 g/mol or less or 800 g/mol or less. In an embodiment, the endgroup is polymeric and has a Mn of 10,000 g/mol or less, 8,000 g/mol or less, 6,000 g/mol or less, or 4,000 g/mol or less. In an embodiment, the endgroup is polymeric and has a Mn of 500 g/mol or more, 1,000 g/mol or more, or 2,000 g/mol or more.

In an embodiment, the endgroup comprises C₂-C₁₆ fluoroalkyl or C₂-C₁₆ fluoroalkyl ether. In an embodiment, the endgroup is formed from a monofunctional alcohol or amine comprising C₂-C₁₆ fluoroalkyl, C₂-C₁₆ fluoroalkyl ether. In an embodiment, the endgroup is formed from 1H,1H-Perfluoro-3,6-dioxaheptan-1-ol, 1H, 1H-Nonafluoro-1-pentanol, 1H,1H-Perfluoro-1-hexyl alcohol, 1H,1H-Perfluoro-3,6,9-trioxadecan-1-ol, 1H,1H-Perfluoro-1-heptyl alcohol, 1H,1H-Perfluoro-3,6-dioxadecan-1-ol, 1H,1H-Perfluoro-1-octyl alcohol, 1H,1H-Perfluoro-1-nonyl alcohol, 1H,1H-Perfluoro-3,6,9-trioxatridecan-1-ol, 1H,1H-Perfluoro-1-decyl alcohol, 1H,1H-Perfluoro-1-undecyl alcohol, 1H,1H-Perfluoro-1-lauryl alcohol, 1H,1H-Perfluoro-1-myristyl alcohol, or 1H,1H-Perfluoro-1-palmityl alcohol.

In an embodiment, the endgroup is present in an amount of at least 0.1 wt%, at least 0.2 wt%, at least 0.3 wt%, or at least 0.5 wt%, based on the total weight of the formulation from which the amphiphilic polyurethane or hydrophobic polyurethane is formed. In an embodiment, the endgroup is present in an amount of at most 3 wt%, at most 2 wt% or at most 1 wt%, based on the total weight of the formulation from which the amphiphilic polyurethane or hydrophobic polyurethane is formed.

### Formation of Polyurethanes

The polyurethanes may be formed as generally known in the art. A catalyst may be employed. In an embodiment, the catalyst is stannous octoate or dibutyltin dilaurate. Amine catalysts may also be used.

### Free Hydrophilic Polymer

In an embodiment, the membrane or composition for forming a membrane comprises a free hydrophilic polymer. A free hydrophilic polymer is a hydrophilic polymer that is not bound to the polyurethane by covalent bonds. In an embodiment, the free hydrophilic polymer comprises poly(ethylene oxide), polyvinylpyrrolidone, polyacrylic acid, polyvinyl alcohol, polyoxazoline, such as a poly(2-methyl-2-oxazoline) or a poly(2-ethyl-2-oxazoline), or hyaluronic acid.

In an embodiment, the free hydrophilic polymer has a number average molecular weight of at least 5,000 g/mol, at least 10,000 g/mol, at least 50,000 g/mol, at least 100,000 g/mol, or at least 200,000 g/mol. In an embodiment, the free hydrophilic polymer has a number average molecular weight of at most 10,000,000 g/mol, at most 5,000,000 g/mol, at most 2,000,000 g/mol, at most 1,000,000 g/mol, at most 500,000 g/mol, or at most 200,000 g/mol.

In an embodiment, the membrane comprises less than 30 wt%. less than 20 wt%, less than 10 wt%, or less than 5 wt% of free hydrophilic polymer, based on the total weight of the membrane. In an embodiment, the composition comprises less than 30 wt%. less than 20 wt%, less than 10 wt%, or less than 5 wt% of free hydrophilic polymer, based on the total weight of the composition excluding any solvent. In an embodiment, the membrane or composition is substantially devoid of or devoid of free hydrophilic polymer.

### Solvent

Compositions comprising a blend of polyurethane also comprise a solvent. To form a membrane, the solvent is evaporated after casting a film from the composition comprising the amphiphilic polyurethane, the hydrophobic polyurethane, optionally the free hydrophilic polymer, and the solvent. In an embodiment, the solvent comprises tetrahydrofuran (THF), methyl-tetrahydrofuran (methyl-THF), dimethylacetamide (DMAc), dimethylformamide (DMF), dimethyl sulfoxide (DMSO), or a mixture thereof. In an embodiment, the solvent comprises tetrahydrofuran (THF) or methyl-tetrahydrofuran (methyl-THF).

A co-solvent may also be present. A co-solvent comprises less than 50 wt% of the total amount of solvent. In an embodiment, a co-solvent is present and is methanol, ethanol, isobutanol, propanol, methyl ethyl ketone, or a mixture thereof. In an embodiment, the solvent comprises 50 wt% or more of tetrahydrofuran (THF), methyl-tetrahydrofuran (methyl-THF), or a mixture thereof and less than 50 wt% of methanol, ethanol, isobutanol, propanol, methyl ethyl ketone, or a mixture thereof.

In an embodiment, the solvent comprises 40 wt% or more of tetrahydrofuran (THF), methyl-tetrahydrofuran (methyl-THF), or a mixture thereof, and methanol, ethanol, isobutanol, propanol, methyl ethyl ketone, or a mixture thereof at an amount of from 1 to 60 wt%, based on the total amount of solvent in the composition.

In an embodiment, the solvent is present in an amount of from 80 to 99.8 wt% of the composition, from 85 wt% to 99.5 wt%, or from 90 wt% to 99 wt%. In an embodiment, the co-solvent is present at less than 50 wt% of the total amount of solvent, less than 40 wt%, less than 30 wt%, less than 20 wt%, or less than 10 wt%. In an embodiment, the solvent comprises at least 40 wt% of THF, methyl-THF, or a mixture thereof, and methanol, ethanol, or a mixture thereof at an amount of from 1 to 60 wt%, based on the total amount of solvent in the composition. In an embodiment, the solvent comprises at least 40 wt% of THF, and methanol, ethanol, or a mixture thereof at an amount of from 1 to 60 wt%, based on the total amount of solvent in the composition. In an embodiment, the solvent comprises at least 70 wt% of THF, and propanol, isobutanol, methyl-THF, or methyl ethyl ketone, or a mixture thereof, at an amount of from 1 to 30 wt%, based on the total amount of solvent in the composition.

### Membranes

Membranes are typically formed by casting the composition comprising the amphiphilic polyurethane, the hydrophobic polyurethane, optionally the free hydrophilic polymer, and the solvent directly onto a substrate, such as a sensor, or onto a support liner. The solvent is then evaporated, optionally by use of vacuum or elevated temperatures. Typical temperatures are from 40 to 90 °C. Additives, such as a mold release agent, may be present to facilitate the casting process. In an embodiment, the composition further comprises a mold release agent, such as ethylene bis(stearamide).

In an embodiment, a membrane is permeable to both glucose and oxygen. In an embodiment, the membrane or a membrane formed from the composition has a glucose transmission rate of from 1×10⁻¹⁰ to 9×10⁻⁹ cm²/sec. In an embodiment, the membrane or a membrane formed from the composition has an oxygen transmission rate of from 1×10⁻⁷ to 1×10⁻² cm²/sec. In an embodiment, the membrane or a membrane formed from the composition has an oxygen transmission rate of from 1×10⁻⁵ to 1×10⁻³ cm²/sec.

In an embodiment, the membrane or composition comprises a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane. In an embodiment, the membrane or composition comprises at least 5 wt%, at least 10 wt%, at least 15 wt%, at least 20 wt%, at least 25 wt%, at least 30 wt%, at least 35 wt%, at least 40 wt%, at least 45 wt%, or at least 50 wt%, based on the total weight of the polyurethane component, of the amphiphilic polyurethane. In an embodiment, the membrane or composition comprises at least 5 wt%, at least 10 wt%, at least 15 wt%, at least 20 wt%, at least 25 wt%, at least 30 wt%, at least 35 wt%, at least 40 wt%, at least 45 wt%, or at least 50 wt%, based on the total weight of the polyurethane component, of the hydrophobic polyurethane. In an embodiment, the membrane or composition comprises at most 95 wt%, at most 90 wt%, at most 85 wt%, at most 80 wt%, at most 75 wt%, at most 70 wt%, at most 65 wt%, at most 60 wt%, at most 55 wt% or at most 50 wt%, based on the total weight of the polyurethane component, of the amphiphilic polyurethane. In an embodiment, the membrane or composition comprises at most 95 wt%, at most 90 wt%, at most 85 wt%, at most 80 wt%, at most 75 wt%, at most 70 wt%, at most 65 wt%, at most 60 wt%, at most 55 wt% or at most 50 wt%, based on the total weight of the polyurethane component, of the hydrophobic polyurethane.

In an embodiment, the membrane has a thickness of from 1 to 100 µm.

In an embodiment, the membrane has a residual solvent content of less than 50 ppm after drying the membrane under nitrogen for 24 hours followed by drying in a convection oven at 50 °C for one hour.

### Applications

The disclosed membranes find utility in medical devices and sensors. Such sensors may detect a wide range of analytes, including glucose, lactic acid, galactose, alcohol, medicinal or recreational drugs, cholesterol, antigens, antibodies, viruses, vitamins, minerals, nutrients, proteins, amino acids, hormones or neurotransmitters. In an embodiment, a sensor for measuring glucose, lactic acid, glutamate, pyruvate, choline, acetylcholine, nitric oxide, sodium, potassium, calcium, chloride, bicarbonate, urea, creatine, or dopamine in the blood stream or another bodily fluid comprises a membrane as disclosed.

In an embodiment, the membrane comprises an enzyme that is reactive with an analyte. In an embodiment, a sensor comprises the membrane and a second membrane, the second membrane comprising an enzyme that is reactive with an analyte.

The medical devices or sensors may be implantable in the body. In an embodiment, a continuous analyte monitoring system comprises the membrane. In an embodiment, a continuous glucose monitoring system comprises the membrane.

The Examples below further elucidate embodiments of the invention, but of course, should not be construed as in any way limiting the scope of the claims.

### Examples

The following test procedures are used in the Examples.

### Residual Solvent

A Gas Chromatograph 7890B using an Agilent CPSil 8 CP, 30m × 0.25 mm × 1 µm column, is used to quantify residual solvent in the polyurethane films. A 0.1 g +/- 0.01 g sample of the film is cut, weighed, and put in a 20 ml scintillation vial. 10 ml of DMSO is added to the scintillation vial and the vial placed on a shaker table until fully dissolved. Once dissolved, 2 ml of the solution is added to a standard GC vial and capped. The vial is placed on a autosampler and run on the GC. The solvent peak area from the sample is used to quantitate residual solvent using a calibration curve.

The GC parameters are shown in Table 0.1, below:

**Table 0.1: Direct Inject Gas Chromatograph Parameters**

| | |
|---|---|
| GC Oven Equilibration Time | 0.5 min |
| Column Max Temperature | 350 °C |
| Slow Fan | Disabled |
| Oven Program | 40 °C for 1.5 min then 20 °C/min to 150 °C for 1 min |
| Run Time | 8 Minutes |
| Post Run Temperature | 120 °C for 0.70 min |

### Oxygen Permeability

Film samples are cut to the appropriate size and masked with foil to seal any leaks. The film thickness is measured. The film is mounted onto a Mocon OxTran 2/20 system and allowed to equilibrate to a constant gas transmission rate, utilizing compressed air as the test gas and 99% nitrogen with 1% hydrogen as the carrier gas. The humidity is 95%. The equilibrium gas transmission rate is recorded and the gas permeability in Barrer is calculated.

### Glucose Diffusivity

A film of the sample is cut in the shape of a circle (diameter ~6 cm) to remove any curled edges or air bubbles. The thickness of the film is measured 10 times using a thickness gauge. The average of the 10 measurements is used as the film thickness. The film is then mounted between two silicone gaskets with vacuum grease. The inner diameter of the gaskets is ~5 cm. The gaskets are then mounted on a Franz cell apparatus with the receptor cavity filled with deionized water and a magnetic stir bar. The receptor vessel is equipped with sampling ports, in which samples can be removed over time. A top is placed on the receptor vessel which seals the film onto the Franz cell and creates the donor vessel. A concentrated glucose solution (2000 mg/dL) is inserted into the donor vessel. The Franz cell is then closed with a lid to prevent evaporation. 100 microliter samples are taken over a period of 6 hours, under stirring at 800 RPM. Deionized water is used to replace the liquid in the donor vessel and maintain sink conditions.

The liquid samples are then analyzed for glucose content using a glucose colorimetric enzyme assay and a UV plate reader. The amount of glucose in each sample is determined using a calibration curve. The slope of mg glucose/cm² over time is determined. This flux value is then utilized to calculate the diffusivity of the glucose of each film. All films are analyzed in at least duplicate to ensure no leaks are present.

### Example 1

A hydrophobic polyurethane is formed from a formulation of 28.14 wt% HMDI, 65.11 wt% poly(tetramethylene oxide) diol having an Mn of approximately 2000 g/mol, 6.48 wt% butanediol, 0.25 wt% 1-octadecanol as endgroups. 0.25 wt% ethylene bis(stearamide) as a mold release agent, and 0.04 wt% stannous octoate as a catalyst (Hydrophobic PU). An amphiphilic polyurethane is formed from a formulation of 33.71 wt% HMDI, 26.42 wt% of poly(tetramethylene oxide) diol having an Mn of approximately 2000 g/mol, 31.59% poly(ethylene glycol) having a Mn of approximately 1450 g/mol, 0.5% mono-methyl-polyethyleneglycol 5000 MW as endgroups, and 8.27 wt% butanediol (Amphiphilic PU).

DSM PurSil^{®} 35 80A is a silicone-containing hydrophobic thermoplastic polyurethane comprising approximately 35 wt% of silicone containing segments. PVP has a Mn of approximately 360,000 g/mol, Mw of approximately 1,000,000 g/mol.

The compositions described in Table 1.1 are formed by thoroughly mixing the ingredients in the stated solvent at 6-10% solids.

**Table 1.1: Compositions**

| Composition | Hydrophobic PU | Amphiphilic PU | DSM PurSil^{®} | PVP | DMAc | THF |
|---|---|---|---|---|---|---|
| A | 4.3 | 4.3 | | | 91.4 | |
| B | 4 | 4 | | | | 92 |
| C | | | 4.77 | 1.43 | 93.8 | |
| D | | | 4.77 | 1.43 | | 93.8 |

Films are cast from the compositions. The films are dried in the specified oven, at the specified temperature, for the specified length of time. The residual solvent is measured. The membrane may be washed with water for 18 hours and the residual solvent measured again. The results are shown in Table 1.2.

**Table 1.2: Results**

| Exp. | Comp. | Oven | Temp. (°C) | Time (hrs) | Water wash | Residual Solvent (ppm) | Glucose Diffusivity (cm²/sec) | Oxygen Permeability (Dk) |
|---|---|---|---|---|---|---|---|---|
| 1-1 | A | Vacuum | 80 | 36 | | 17,237 | 8.57×10⁻⁹ | 13 |
| 1-2 | A | Convection | 80 | 16 | | 105 | 8.57×10⁻⁹ | 13 |
| 1-3 | A | Convection | 80 | 16 | X | 91 | 8.57×10⁻⁹ | 13 |
| 1-4 | B | Convection | 50 | 16 | | 0 | 8.57×10⁻⁹ | 11.2 |
| 1-5 | C | Convection | 50 | 16 | | 106 | 2.05×10⁻⁹ | 27.3 |
| 1-6 | C | Convection | 50 | 16 | X | 81 | 2.05×10⁻⁹ | 27.3 |

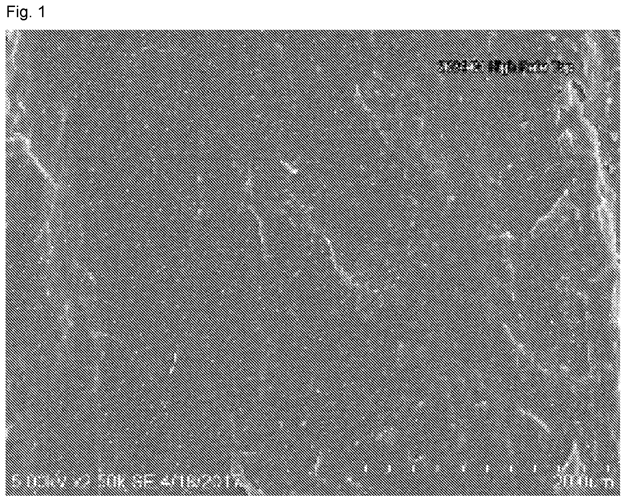

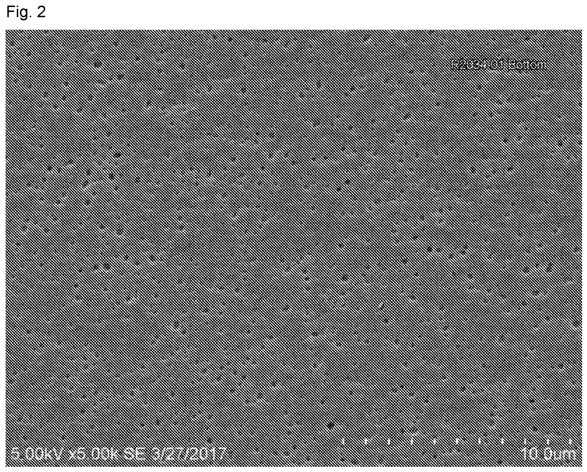

A membrane formed from Experiment 1-4 is shown in Fig. 1. The membrane shows good isotropy and no visible pinholes. A membrane formed from Experiment 1-5 is shown in Fig. 2. The membrane shows numerous undesirable pinholes.

The membrane comprising a blend of amphiphilic polyurethane and hydrophobic polyurethane can be cast in THF under less harsh conditions. Furthermore, no residual solvent is found in the film, even before water washing.

### Example 2

The solubility of various polyurethane formulations is investigated. The polyurethanes and the free hydrophilic polymer are the same as used in Example 1. The solubility is tested at room temperature.

**Table 2-1**

| Ex. | Polyurethanes | | Free Hydrophilic polymer | Solvent | Solubility | %Solids |
|---|---|---|---|---|---|---|
| 2-1 | Amphiphilic PU | Hydrophobic PU | | DMAc | Yes | 6.54 |
| 2-2 | Amphiphilic PU | Hydrophobic PU | | THF | Yes | 6.57 |
| 2-3 | Amphiphilic PU | DSM PurSil^{®} | | DMAc | No | |
| 2-4 | Amphiphilic PU | DSM PurSil^{®} | | THF | No | |
| 2-5 | Amphiphilic PU | - | PVP | DMAc | Yes | 6.14 |
| 2-6 | Amphiphilic PU | - | PVP | THF | Yes, but not all PVP went into solution | 4.74 |
| 2-7 | DSM PurSil^{®} | - | PVP | DMAc | No | |
| 2-8 | DSM PurSil^{®} | - | PVP | THF | No | |

The glucose diffusivity and oxygen permeability numbers are also determined for the compositions that exhibited some degree of solubility, with the exception of 2-5. Example 2-5 was the victim of a measurement error; reliable data for glucose diffusivity was not obtained.

**Table 2-2**

| Ex. | Glucose Diffusivity (cm²/sec) | Oxygen Permeability (Dk) |
|---|---|---|
| 2-1 | 2.93 × 10⁻⁹ | 21.7 |
| 2-2 | 8.00 × 10⁻¹⁰ | 22.7 |
| 2-5 | Measurement Error | 34.7 |
| 2-6 | 9.59 × 10⁻¹² | 38 |

Only the blend of the amphiphilic polyurethane and hydrophobic polyurethane shows suitable solubility in THF.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the claims unless otherwise stated. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. While certain optional features are described as embodiments of the invention, the description is meant to encompass and specifically disclose all combinations of these embodiments unless specifically indicated otherwise or physically impossible.

## Claims

1. A method of forming a membrane comprising the steps of forming a film from a composition comprising a polyurethane component and a solvent, and evaporating the solvent thereby forming a membrane,
wherein the composition comprises from 0.5 wt% to 10 wt% of the polyurethane component and from 90 to 99.5 wt% of the solvent, the polyurethane component comprising a blend of from 5 wt% to 95 wt%, based on the total weight of the polyurethane component, of an amphiphilic polyurethane, and from 5 wt% to 95 wt%, based on the total weight of the polyurethane component, of a hydrophobic polyurethane, wherein the amphiphilic polyurethane comprises a first amphiphilic polyurethane or a second amphiphilic polyurethane, and
a. the first amphiphilic polyurethane comprises the reaction product of:
i. an aliphatic diisocyanate;
ii. a hydrophilic polymer diol;
iii. a hydrophobic poly(alkylene oxide) diol; and
iv. a chain extender;
b. the second amphiphilic polyurethane comprises the reaction product of:
i. an aliphatic diisocyanate;
ii. a copolymer diol comprising a hydrophilic polymer and a hydrophobic poly(alkylene oxide); and
iii. a chain extender;
c. the hydrophobic polyurethane comprises the reaction product of:
i. an aliphatic diisocyanate;
ii. a hydrophobic poly(alkylene oxide) diol; and
iii. a chain extender;
wherein the amphiphilic polyurethane and the hydrophobic polyurethane are substantially devoid of siloxane moieties,
wherein the solvent comprises tetrahydrofuran (THF), methyl-tetrahydrofuran (methyl-THF), or a mixture thereof, and
wherein the membrane has a residual solvent content of less than 50 ppm after drying the membrane in a convection oven at 50 °C for 16 hours after forming the membrane.

2. The method of claim 1, wherein the amphiphilic polyurethane comprises from 30 wt% to 85 wt%, based on the total weight of the amphiphilic polyurethane, of the residue of a hydrophilic poly(alkylene oxide) diol, a hydrophobic poly(alkylene oxide) diol, or a copolymer diol comprising a hydrophilic poly(alkylene oxide) and a hydrophobic poly(alkylene oxide).

3. The method of claim 1 or 2, wherein the amphiphilic polyurethane, the hydrophobic polyurethane, or both are linear and further comprise an endgroup at each terminus of the backbone.

4. The method of any one of claims 1-3, wherein the amphiphilic polyurethane is linear and comprises an endgroup, and the endgroup comprises poly(ethylene oxide).

5. The method of any one of claims 1-4, wherein the amphiphilic polyurethane, the hydrophobic polyurethane, or both further comprise an endgroup and the endgroup comprises C₂-C₂₀ alkyl, C₂-C₁₆ fluoroalkyl, or C₂-C₁₆ fluoroalkyl ether.

6. The method of any one of claims 1-5, wherein the composition or membrane are substantially devoid of free hydrophilic polymer.

7. The method of any one of claims 1-6, wherein the amphiphilic polyurethane comprises the first amphiphilic polyurethane and the first amphiphilic polyurethane comprises the residue of a hydrophilic poly(alkylene oxide) diol.

8. The method of any one of claims 1-6, wherein the amphiphilic polyurethane comprises the second amphiphilic polyurethane and the second amphiphilic polyurethane comprises the residue of copolymer diol comprising a hydrophilic poly(alkylene oxide) and a hydrophobic poly(alkylene oxide).

9. The method of any one of claims 1-8, wherein the polyurethane component comprises a blend of from 35 wt% to 65 wt%, based on the total weight of the polyurethane component, of the amphiphilic polyurethane, and from 35 wt% to 65 wt%, based on the total weight of the polyurethane component, of the hydrophobic polyurethane.

10. The method of any one of claims 1-9, wherein the hydrophobic polyurethane is devoid of polyethylene oxide and polyoxazoline.

11. The method of any one of claims 1-10, wherein the amphiphilic polyurethane consists of the reaction product of:
i. an aliphatic diisocyanate;
ii. a hydrophilic polymer diol;
iii. a hydrophobic poly(alkylene oxide) diol; and
iv. a chain extender.

12. The method of any one of claims 1-11, wherein the backbone of the hydrophobic polyurethane consists of the reaction product of:
i. an aliphatic diisocyanate;
ii. a hydrophobic poly(alkylene oxide) diol; and
iii. a chain extender.

13. The method of any one of claims 1-12, wherein the hydrophobic polymer diol consists of poly(propylene oxide) diol, poly(tetramethylene oxide) diol, a copolymer diol comprising poly(propylene oxide) and poly(tetramethylene oxide), or a mixture thereof.

14. The method of any one of claims 1-13, wherein the solvent consists of tetrahydrofuran (THF), methyl-tetrahydrofuran (methyl-THF), or a mixture thereof.

15. The method according to any one of claims 1-13, wherein the solvent consists of at least 50 wt% of THF, methyl-THF, or a mixture thereof, and optionally, methanol, ethanol, isobutanol, propanol, methyl ethyl ketone, or a mixture thereof at an amount of 50 wt% or less, based on the total amount of solvent in the composition.

16. A membrane formed from the method according to any one of claims 1-15.

17. A sensor comprising the membrane of claim 16, wherein the sensor is configured to detect glucose, lactic acid, glutamate, pyruvate, choline, acetylcholine, nitric oxide, sodium, potassium, calcium, chloride, bicarbonate, urea, creatine, or dopamine in the blood stream or another bodily fluid.

## Patentansprüche

1. Verfahren zur Herstellung einer Membran, umfassend die Schritte des Bildens eines Films aus einer Zusammensetzung, die eine Polyurethankomponente und ein Lösungsmittel umfasst, und Verdunsten des Lösungsmittels, um eine Membran zu bilden,
wobei die Zusammensetzung von 0,5 Gew.-% bis 10 Gew.-% an der Polyurethankomponente und von 90 bis 99,5 Gew.-% an dem Lösungsmittel umfasst, wobei die Polyurethankomponente ein Gemisch von 5 Gew.-% bis 95 Gew.-%, bezogen auf das Gesamtgewicht der Polyurethankomponente, an einem amphiphilen Polyurethan und von 5 Gew.-% bis 95 Gew.-%, bezogen auf das Gesamtgewicht der Polyurethankomponente, an einem hydrophoben Polyurethan umfasst, wobei das amphiphile Polyurethan ein erstes amphiphiles Polyurethan oder ein zweites amphiphiles Polyurethan umfasst, und
a. das erste amphiphile Polyurethan das Reaktionsprodukt von:
i. einem aliphatischen Diisocyanat;
ii. einem hydrophilen Polymerdiol;
iii. einem hydrophoben Poly(alkylenoxid)diol; und
iv. einem Kettenverlängerungsmittel;
umfasst;
b. das zweite amphiphile Polyurethan das Reaktionsprodukt von:
i. einem aliphatischen Diisocyanat;
ii. einem Copolymerdiol, das ein hydrophiles Polymer und ein hydrophobes Poly(alkylenoxid) umfasst; und
iii. einem Kettenverlängerungsmittel;
umfasst;
c. das hydrophobe Polyurethan das Reaktionsprodukt von:
i. einem aliphatischen Diisocyanat;
ii. einem hydrophoben Poly(alkylenoxid)diol; und
iii. einem Kettenverlängerungsmittel;
umfasst;
wobei das amphiphile Polyurethan und das hydrophobe Polyurethan im Wesentlichen frei von Siloxaneinheiten sind,
wobei das Lösungsmittel Tetrahydrofuran (THF), Methyltetrahydrofuran (Methyl-THF) oder ein Gemisch davon umfasst und
wobei die Membran einen Restlösungsmittelgehalt von weniger als 50 ppm nach 16 Stunden Trocknen der Membran in einem Konvektionsofen bei 50 °C nach dem Bilden der Membran aufweist.

2. Verfahren gemäß Anspruch 1, wobei das amphiphile Polyurethan von 30 Gew.-% bis 85 Gew.-%, bezogen auf das Gesamtgewicht des amphiphilen Polyurethans, an dem Rest eines hydrophilen Poly(alkylenoxid)diols, eines hydrophoben Poly(alkylenoxid)diols oder eines Copolymerdiols umfassend ein hydrophiles Poly(alkylenoxid) und ein hydrophobes Poly(alkylenoxid) umfasst.

3. Verfahren gemäß Anspruch 1 oder 2, wobei das amphiphile Polyurethan, das hydrophobe Polyurethan oder beide linear sind und ferner eine Endgruppe an jedem Ende des Gerüsts umfassen.

4. Verfahren gemäß einem der Ansprüche 1-3, wobei das amphiphile Polyurethan linear ist und eine Endgruppe umfasst und die Endgruppe Poly(ethylenoxid) umfasst.

5. Verfahren gemäß einem der Ansprüche 1-4, wobei das amphiphile Polyurethan, das hydrophobe Polyurethan oder beide ferner eine Endgruppe umfassen und die Endgruppe C₂-C₂₀Alkyl, C₂-C₁₆Fluoralkyl oder C₂-C₁₆Fluoralkylether umfasst.

6. Verfahren gemäß einem der Ansprüche 1-5, wobei die Zusammensetzung oder Membran im Wesentlichen frei von hydrophilem Polymer sind.

7. Verfahren gemäß einem der Ansprüche 1-6, wobei das amphiphile Polyurethan das erste amphiphile Polyurethan umfasst und das erste amphiphile Polyurethan den Rest eines hydrophilen Poly(alkylenoxid)diols umfasst.

8. Verfahren gemäß einem der Ansprüche 1-6, wobei das amphiphile Polyurethan das zweite amphiphile Polyurethan umfasst und das zweite amphiphile Polyurethan den Rest von Copolymerdiol umfassend ein hydrophiles Poly(alkylenoxid) und ein hydrophobes Poly(alkylenoxid) umfasst.

9. Verfahren gemäß einem der Ansprüche 1-8, wobei die Polyurethankomponente ein Gemisch von 35 Gew.-% bis 65 Gew.-%, bezogen auf das Gesamtgewicht der Polyurethankomponente, an dem amphiphilen Polyurethan und von 35 Gew.-% bis 65 Gew.-%, bezogen auf das Gesamtgewicht der Polyurethankomponente, an dem hydrophoben Polyurethan umfasst.

10. Verfahren gemäß einem der Ansprüche 1-9, wobei das hydrophobe Polyurethan frei von Polyethylenoxid und Polyoxazolin ist.

11. Verfahren gemäß einem der Ansprüche 1-10, wobei das amphiphile Polyurethan aus dem Reaktionsprodukt von:
i. einem aliphatischen Diisocyanat;
ii. einem hydrophilen Polymerdiol;
iii. einem hydrophoben Poly(alkylenoxid)diol; und
iv. einem Kettenverlängerungsmittel;
besteht.

12. Verfahren gemäß einem der Ansprüche 1-11, wobei das Gerüst des hydrophoben Polyurethans aus dem Reaktionsprodukt von:
i. einem aliphatischen Diisocyanat;
ii. einem hydrophoben Poly(alkylenoxid)diol; und
iii. einem Kettenverlängerungsmittel;
besteht.

13. Verfahren gemäß einem der Ansprüche 1-12, wobei das hydrophobe Polymerdiol aus Poly(propylenoxid)diol, Poly(tetramethylenoxid)diol, einem Copolymerdiol umfassend Poly(propylenoxid) und Poly(tetramethylenoxid) oder einem Gemisch davon besteht.

14. Verfahren gemäß einem der Ansprüche 1-13, wobei das Lösungsmittel aus Tetrahydrofuran (THF), Methyltetrahydrofuran (Methyl-THF) oder ein Gemisch davon besteht.

15. Verfahren gemäß einem der Ansprüche 1-13, wobei das Lösungsmittel aus wenigstens 50 Gew.-% THF, Methyl-THF oder einem Gemisch davon und gegebenenfalls Methanol, Ethanol, Isobutanol, Propanol, Methylethylketon oder einem Gemisch davon in einer Menge von 50 Gew.-% oder weniger, bezogen auf die Gesamtmenge an Lösungsmittel in der Zusammensetzung, besteht.

16. Membran, gebildet durch das Verfahren gemäß einem der Ansprüche 1-15.

17. Sensor, umfassend die Membran gemäß Anspruch 16, wobei der Sensor dafür gestaltet ist, Glucose, Milchsäure, Glutamat, Pyruvat, Cholin, Acetylcholin, Stickstoffmonoxid, Natrium, Kalium, Calcium, Chlorid, Bicarbonat, Harnstoff, Kreatin oder Dopamin in dem Blutstrom oder einem anderen Körperfluid zu erfassen.

## Revendications

1. Procédé de formation d'une membrane comprenant les étapes de formation d'un film à partir d'une composition comprenant un composant de type polyuréthane et un solvant, et l'évaporation du solvant, formant ainsi une membrane,
la composition comprenant de 0,5 % en poids à 10 % en poids du composant de type polyuréthane et de 90 à 99,5 % en poids du solvant, le composant de type polyuréthane comprenant un mélange allant de 5 % en poids à 95 % en poids, sur la base du poids total du composant de type polyuréthane, d'un polyuréthane amphiphile, et de 5 % en poids à 95 % en poids, sur la base du poids total du composant de type polyuréthane, d'un polyuréthane hydrophobe, le polyuréthane amphiphile comprenant un premier polyuréthane amphiphile ou un deuxième polyuréthane amphiphile, et
a. le premier polyuréthane amphiphile comprenant le produit de réaction de :
i. un diisocyanate aliphatique ;
ii. un polymère diol hydrophile ;
iii. un poly(oxyde d'alkylène) diol hydrophobe ; et
iv. un extenseur de chaînes ;
b. le deuxième polyuréthane amphiphile comprenant le produit de réaction de :
i. un diisocyanate aliphatique ;
ii. un diol copolymérique comprenant un polymère hydrophile et un poly(oxyde d'alkylène) hydrophobe ; et
iii. un extenseur de chaînes ;
c. le polyuréthane hydrophobe comprenant le produit de réaction de :
i. un diisocyanate aliphatique ;
ii. un poly(oxyde d'alkylène) diol hydrophobe ; et
iii. un extenseur de chaînes ;
le polyuréthane amphiphile et le polyuréthane hydrophobe étant sensiblement exempts de groupements siloxane,
le solvant comprenant le tétrahydrofuranne (THF), le méthyl-tétrahydrofuranne (méthyl-THF) ou un mélange correspondant, et
la membrane présentant une teneur en solvant résiduel inférieur à 50 ppm après séchage de la membrane dans un four à convection à 50 °C pendant 16 heures après formation de la membrane.

2. Procédé selon la revendication 1, le polyuréthane amphiphile comprenant de 30 % en poids à 85 % en poids, sur la base du poids total du polyuréthane amphiphile, du radical d'un poly(oxyde d'alkylène) diol hydrophile, d'un poly(oxyde d'alkylène) diol hydrophobe ou d'un diol copolymérique comprenant un poly(oxyde d'alkylène) hydrophile et un poly(oxyde d'alkylène) hydrophobe.

3. Procédé selon la revendication 1 ou 2, le polyuréthane amphiphile, le polyuréthane hydrophobe ou les deux étant linéaire(s) et comprenant en outre un groupe terminal au niveau de chaque terminaison du squelette.

4. Procédé selon l'une quelconque des revendications 1 à 3, le polyuréthane amphiphile étant linéaire et comprenant un groupe terminal, et le groupe terminal comprenant un poly(oxyde d'éthylène).

5. Procédé selon l'une quelconque des revendications 1 à 4, le polyuréthane amphiphile, le polyuréthane hydrophobe ou les deux comprenant un groupe terminal et le groupe terminal comprenant C₂₋₂₀ alkyle, C₂₋₁₆ fluoroalkyle ou C₂₋₁₆ fluoroalkyl-éther.

6. Procédé selon l'une quelconque des revendications 1 à 5, la composition ou la membrane étant sensiblement exempte de polymère hydrophile libre.

7. Procédé selon l'une quelconque des revendications 1 à 6, le polyuréthane amphiphile comprenant le premier polyuréthane amphiphile et le premier polyuréthane amphiphile comprenant le radical d'un poly(oxyde d'alkylène) diol hydrophile.

8. Procédé selon l'une quelconque des revendications 1 à 6, le polyuréthane amphiphile comprenant le deuxième polyuréthane amphiphile et le deuxième polyuréthane amphiphile comprenant le radical d'un diol copolymérique comprenant un poly(oxyde d'alkylène) hydrophile et un poly(oxyde d'alkylène) hydrophobe.

9. Procédé selon l'une quelconque des revendications 1 à 8, le composant de type polyuréthane comprenant un mélange allant de 35 % en poids à 65 % en poids, sur la base du poids total du composant de type polyuréthane, du polyuréthane amphiphile, et de 35 % en poids à 65 % en poids, sur la base du poids total du composant de type polyuréthane, du polyuréthane hydrophobe.

10. Procédé selon l'une quelconque des revendications 1 à 9, le polyuréthane hydrophobe étant exempt de poly(oxyde d'éthylène) et de polyoxazoline.

11. Procédé selon l'une quelconque des revendications 1 à 10, le polyuréthane amphiphile étant constitué du produit de réaction de :
i. un diisocyanate aliphatique ;
ii. un polymère diol hydrophile ;
iii. un poly(oxyde d'alkylène) diol hydrophobe ; et
iv. un extenseur de chaînes.

12. Procédé selon l'une quelconque des revendications 1 à 11, le squelette du polyuréthane hydrophobe étant constitué du produit de réaction de :
i. un diisocyanate aliphatique ;
ii. un poly(oxyde d'alkylène) diol hydrophobe ; et
iii. un extenseur de chaînes.

13. Procédé selon l'une quelconque des revendications 1 à 12, le polymère diol hydrophobe étant constitué d'un poly(oxyde de propylène) diol, d'un poly(oxyde de tétraméthylène) diol, d'un copolymère diol comprenant un poly(oxyde de propylène) et un poly(oxyde de tétraméthylène), ou un mélange correspondant.

14. Procédé selon l'une quelconque des revendications 1 à 13, le solvant étant constitué de tétrahydrofuranne (THF), de méthyl-tétrahydrofuranne (méthyl-THF) ou d'un mélange correspondant.

15. Procédé selon l'une quelconque des revendications 1 à 13, le solvant étant constitué d'au moins 50 % en poids de THF, de méthyl-THF ou d'un mélange correspondant, et éventuellement, de méthanol, d'éthanol, d'isobutanol, de propanol, de méthyléthylcétone ou d'un mélange correspondant à raison d'une quantité de 50 % en poids ou moins, sur la base de la quantité totale de solvant dans la composition.

16. Membrane formée à partir du procédé selon l'une quelconque des revendications 1 à 15.

17. Capteur comprenant la membrane selon la revendication 16, le capteur étant configuré pour détecter le glucose, l'acide lactique, un glutamate, un pyruvate, la choline, l'acétylcholine, l'oxyde nitrique, le sodium, le potassium, le calcium, un chlorure, un bicarbonate, l'urée, la créatine ou la dopamine dans le flux sanguin ou un autre fluide corporel.
